# EUROPEAN PATENT APPLICATION

(11) **EP 1 595 835 A1**
(43) Date of publication of application: **16.11.2005**
(21) Application number: 04702065.6
(22) Date of filing: 14.01.2004
(51) Int. Cl.: B65H 23/038, A61F 13/15

(54) **METHOD AND APPARATUS FOR GUIDING SIDE EDGES OF CONTINUOUSLY RUNNING WEB**

(30) Priority: 14.01.2003 JP 2003005961
(71) Applicant: UNI-CHARM CO., LTD., Kawanoe-shi Ehime 799-0111 (JP)
(72) Inventor: NOMURA, Hironori c/o Technical Center Uni-Charm CO, toyo-gun Kagawa 769-1602 (JP); SHIMAKAWA, Taiji c/o Technical Center Uni-Charm CO, toyo-gun Kagawa 769-1602 (JP); YAMAMOTO, Hiroki c/o Technical Center Uni-Charm CO, toyo-gun Kagawa 769-1602 (JP)
(74) Representative: Sperling, Rüdiger
(86) International application number: PCT/JP2004/000180
(87) International publication number: WO 2004/063069

(57) **Abstract**

A roll means comprising a first nip roll (61) rotating about a shaft (66) extending in a direction defined between a machine direction (MD) and a cross direction (CD) is put in contact with a side edge (31) of a web (21) continuously running in the machine direction (MD). The shaft (66) is mounted on an arm (49) adapted to rotate back-and-forth so that the shaft (66) can swing in- and outward in the cross direction (CD). Downstream of the roll means, the side edge (31) is position-detected and the arm (49) is made swing by a desired angle determined on the basis of a difference between the actual position and a desired position of the side edge (31).

## Description

### TECHNICAL FIELD

The present invention relates to a method and an apparatus allowing, in the course of continuously feeding a flexible web of nonwoven fabric, plastic film, paper or the like in a machine direction, side edges of such a web to be guided to desired positions in a cross direction orthogonal to the machine direction.

### BACKGROUND OF THE INVENTION

It is well known in prior art references 1, 2, 3 listed below, in the course of continuously feeding a flexible web of nonwoven fabric, plastic film, paper or the like in a machine direction, to put guide roll means rotating about respective shafts each obliquely extending at a desired angle with respect to the machine direction in contact with the vicinities of side edges of such a web and thereby to move the side edges so that the web may be broadened. It is also well known that the web can be unwrinkled by guiding the side edges of the web. Prior art reference discloses a method for guiding the side edges of the web continuously fed to desired positions by operatively associating such guide roll means with detector means adapted to detect actual positions of the web.
Prior art reference 1: GB Patent Publication No. 1,373,682
Prior art reference 2: US Patent Publication No. 4,920,622
Prior art reference 3: US Patent Publication No. 2,718,046

Of the apparatuses disclosed in Prior art references 1 through 3, one apparatus has the guide roll means fixed at a predetermined angle relative to the machine direction, another apparatus is adapted to vary a contact pressure between the guide roll means and the web, and further another apparatus has the guide roll means in the form of paired nip rolls adapted to vary a nip pressure against the web. When it is intended to guide the side edges of the web detected to be out of coincidence with desired positions to these desired positions by appropriately varying the contact pressure or the nip pressure, it is relatively easy to shift the side edges outward in the axial direction of the guide roll means, i.e., outward in the cross direction corresponding to the transverse direction of the web. However, it is difficult to shift the side edges in the directions other than the outward direction, for example, inward in the cross direction. For these apparatuses disclosed in Prior art references 1, 2, 3 respectively provided with such guide roll means, it has been impossible to guide the side edges of the web little by little with a high accuracy or to position-shift the side edges of the web quickly. In addition, the web of nonwoven fabric, plastic film or the like which has been tightly wounded around a roll core generally exhibits a subtle variation in a width of the web as the web is released and fed in the machine direction directly from such tightly wound state. Such subtle variation of the width of the web may occur also due to a pulling force exerted on the web in the machine direction. Synergistic effect of these variation destabilizes the width of the web and therefore destabilizes positions of the side edges of the web in the cross direction. In consequence, it is difficult to continuously make articles each having a high dimensional accuracy by utilizing the side edges of the web.

It is an object of the present invention to provide a method and an apparatus facilitating the side edges of the web continuously running in the machine direction to be quickly guided to desired positions in the cross direction when these side edges are detected to be out of coincidence with the desired positions in the cross direction.

### DISCLOSURE OF THE INVENTION

According to a first aspect of the present invention, there is provided a method for guiding a web continuously running in a machine direction and having a pair of side edges opposed to each other in a cross direction orthogonal to the machine direction so that at least one of the side edges is guided to a predetermined position in the cross direction by putting roll means rotating about a shaft extending in a direction defined between the machine direction and the cross direction in contact with a vicinity of at least one of the side edges.

The first aspect of the present invention comprises the steps of: mounting an arm having a proximal end and a distal end opposed to the proximal end at the proximal end on a pivot pin adapted to rotate back-and-forth so that the arm swings about the pivot pin in- and outward as viewed in the cross direction, supporting the shaft of the roll means by the distal end of the arm so that the roll means also swing about the pivot pin in- and outward, detecting, at downstream of the roll means, a position of the one side edge immediately after this side edge has been guided, and driving the arm by driving means adapted to operate on the basis of a difference between the position detected in this manner and the predetermined position so that the arm swings in- or outward in the cross direction by a predetermined angle.

The present invention on the first aspect may be implemented also in manners as follow:
(1) The driving means comprises an extensible/retractable arm operatively associated with a servomotor adapted to rotate in a direction and at a rotational frequency depending on the difference between the detected position and the predetermined position and thereby to extend or retract the extensible/retractable arm so that the side edge is guided to the predetermined position.
(2) The roll means is put in contact with at least one side of the web.
(3) The roll means comprises a pair of rolls extending in parallel to each other and adapted to nip at least one side edge and a vicinity thereof.
(4) The roll means are provided in vicinities of a pair of the side edges to guide these side edges to the respective predetermined positions.

According to a second aspect of the present invention, there is provided an apparatus for guiding a web continuously running in a machine direction and having a pair of side edges opposed to each other in a cross direction orthogonal to the machine direction so that at least one of the side edges is guided to a predetermined position in the cross direction by putting roll means rotating about a shaft extending in a direction defined between the machine direction and the cross direction in contact with a vicinity of at least one of the side edges.

The second aspect of the present invention further comprises the following features: the shaft of the roll means is supported on a distal end of an arm having a proximal end and the distal end opposed to the proximal end; the proximal end of the arm is mounted on a pivot pin adapted to rotate back-and-forth so that the arm swings about the pivot pin in- and outward as viewed in the cross direction; detector means is provided downstream of the roll means in the machine direction to detect a position of the one side edge after this side edge has been guided; and the arm is operatively associated with driving means adapted to operate on the basis of a difference between the position detected by the detector means and the predetermined position so that the arm swings in- or outward in the cross direction by a predetermined angle.

The second aspect of the present invention may be implemented also in manners as follow:
(1) The driving means comprises an extensible/retractable arm operatively associated with a servomotor.
(2) The roll means is put in contact with at least one side of the web.
(3) The roll means comprises a pair of rolls extending in parallel to each other and adapted to nip at least one side edge and a vicinity thereof.
(4) The roll means are provided in vicinities of a pair of the side edges to guide these side edges to the respective desired positions.

### BRIEF DESCRIPTION OF THE DRAWINGS

Fig. 1 is a plan view showing a partially cutaway disposable diaper;
Fig. 2 is a diagram illustrating a part of process for making the disposable diaper;
Fig. 3 is a diagram illustrating a part of Fig. 2 in an enlarged scale;
Fig. 4 is a sectional view taken along the line IV-IV in Fig. 3;
Fig. 5 is a sectional view taken along the line V-V in Fig. 3;
Fig. 6 is a diagram illustrating a part of Fig. 5 in an enlarged scale;
Fig. 7 is a sectional view taken along the line VII-VII in Fig. 5;
Fig. 8 is a sectional view taken along the line VIII-VIII in Fig. 5;
Fig. 9 is a view similar to Fig. 3, illustrating one preferred embodiment of the present invention; and
Fig. 10 is a view similar to Fig. 4, illustrating this embodiment.

### DESCRIPTION OF THE BEST MODE FOR WORKING OF THE INVENTION

Details of a method and an apparatus according to the present invention will be more fully understood from the description given hereunder with reference to the accompanying drawings.

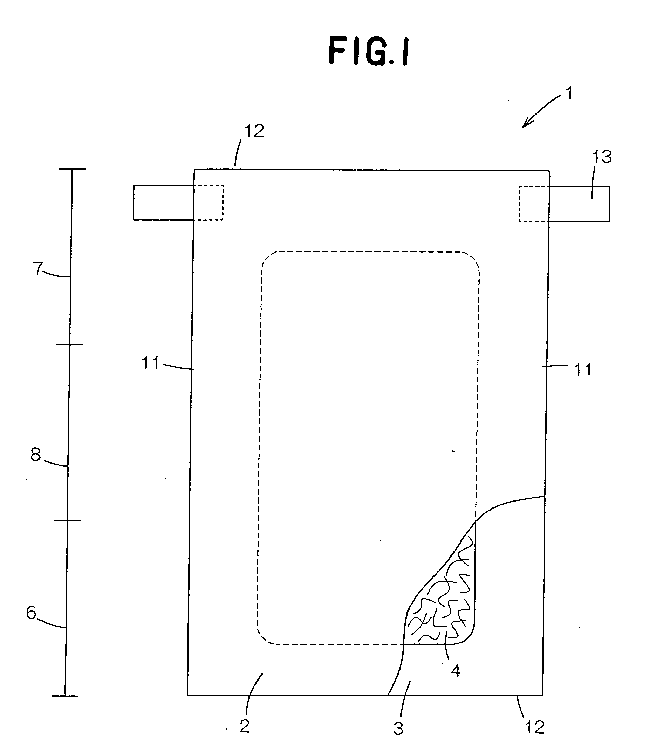

A disposable diaper 1 shown by Fig. 1 in a plan view as partially broken away is a product obtained by a method and an apparatus according to the present invention. This diaper 1 comprises a liquid-pervious topsheet 2, a liquid-impervious backsheet 3 and a liquid-absorbent core 4 interposed between these sheets 2, 3. The diaper 1 has a in- and outward direction (longitudinal direction as viewed in Fig. 1) and a waist-surrounding direction (transverse direction as viewed in Fig. 1). The diaper 1 is peripherally contoured by a pair of side edges 11 extending parallel to each other in the in- and outward direction and a pair of ends extending parallel to each other in the waist-surrounding direction. As viewed in the in- and outward direction, the diaper 1 is composed of a front waist region 6, a rear waist region 7 and a crotch region 8 extending between these waist regions 6, 7 wherein tape fasteners 13 laterally extend from the side edges 11 in the rear waist region 7. The topsheet 2 may be formed from liquid-pervious materials such as nonwoven fabrics or aperture plastic films, the backsheet 3 may be formed from liquid-impervious materials selected from plastic films, nonwoven fabrics and plastic film/nonwoven fabric laminates. The diaper 1 further comprises elastic members attached in a stretched state thereto so as to extend along the side edges 11 and the ends 12.

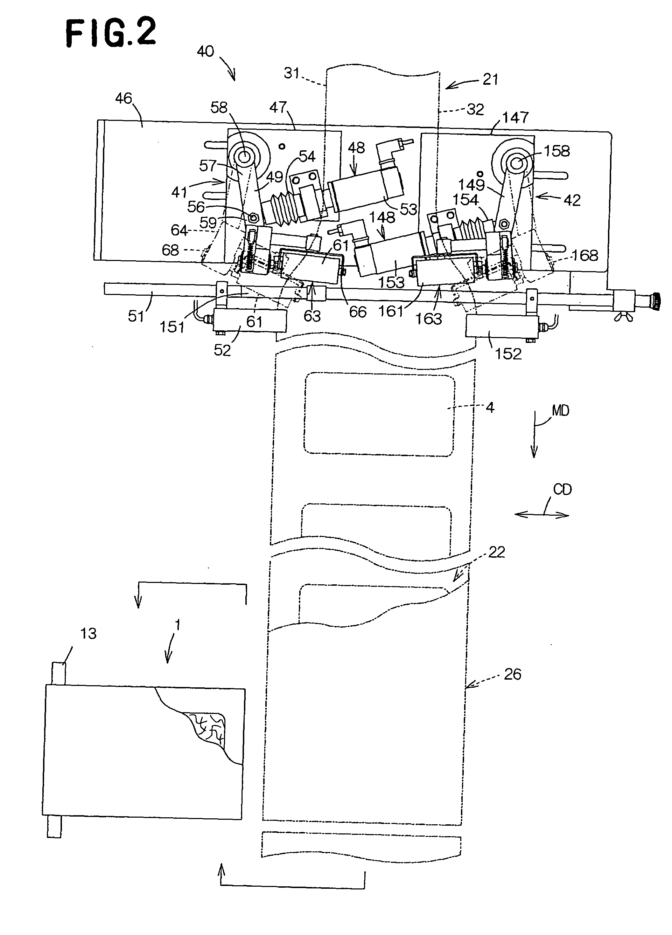

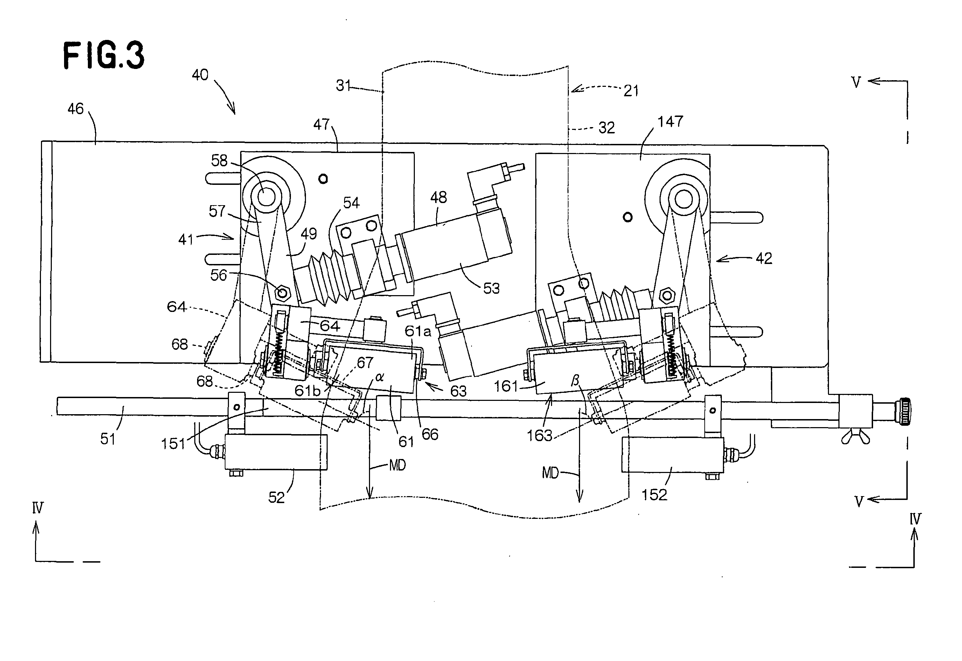

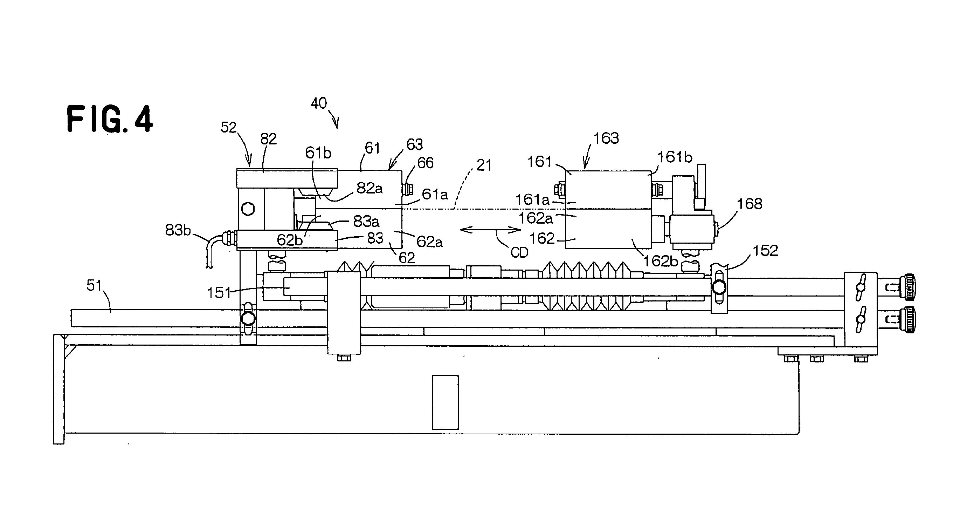

Fig. 2 is a plan view illustrating a part of process for continuously making the diaper 1, Fig. 3 is a scale-enlarged plan view illustrating a part of Fig. 2 and Fig. 4 is a partially cutaway sectional view taken along the line IV-IV in Fig. 3. As viewed in Fig. 2, a first continuous web 21 destined to form the backsheet 3 of the diaper 1 is continuously fed in a machine direction MD downward from above. The continuous web 21 is guided through an edge controller 40 to control a width of the web 21 to a desired dimension and then loaded thereon with the cores 4 which are arranged intermittently in the machine direction MD. Then, second continuous web 22 destined to form the topsheet 2 of the diaper 1 is continuously fed so as to cover the cores 4 whereupon the first continuous web 21 and the second continuous web 22 are joined together around the respective cores 4 by means of adhesives or a suitable welding technique to form a continuous assembly 26. The continuous assembly 26 is cut along lines each extending in a cross direction CD orthogonal to the machine direction MD between each pair of the adjacent cores 4 followed by attaching a pair of the tape fasteners 13 to the first web 21 of each unit assembly cut from the continuous assembly 26. In this manner, the individual diapers 1 as shown by Fig. 1 are obtained. In Figs. 2, 3 and 4, the first and second continuous webs 21, 22 as well as the cores 4 are indicated by imaginary lines and, in Fig. 2, the diaper 1 is illustrated as partially broken away.

The first continuous web 21 has first and second side edges 31, 32 opposed to each other in the cross direction CD and extending parallel to each other in the machine direction MD. The edge controller 40 functions as a controlling means for the first continuous web 21. Specifically, the edge controller 40 guides at least one of vicinities of the first side edge 31 and the second side edge 32 to a position predetermined position in the cross direction CD and thereby controls the width of the first continuous web 21 to a desired dimension. The edge controller 40 exemplarily illustrated comprises a first edge controller 41 operatively associated with the first side edge 31 and a second edge controller 42 operatively associated with the second side edge 32. These two edge controllers 41, 42 are substantially identical to each other, so the first edge controller 41 will be primarily described with reference to Figs. 2 through 4. The term used herein "vicinities of the respective side edges" should be understood to include the respective side edges and the vicinities thereof.

The first edge controller 41 includes a movable plate 47 adapted to be movable in the cross direction CD relative to a base plate 46 extending across the first continuous web 21. The movable plate 47 is provided with an actuator 48 and a swing arm 49. A support bar 51 mounted on the base plate 46 downstream in the machine direction MD and extending in the cross direction CD is provided with a photoelectric sensor unit 52. The photoelectric sensor unit 52 electrically connected with the actuator 48 functions to detect an actual position of the first side edge of the first continuous web 21 and to compare this actual position to a predetermined position on which the first side edge 31 should fall. Based on such comparison, the photoelectric sensor unit 52 calculates a direction and a distance in and by which the first side edge 31 should be shifted to the predetermined position. The actuator 48 comprising a servomotor 53, a extensible/retractable arm 54 and a ball screw joint (not shown) interposed between these motor 53 and the arm 54 rotates the motor 53 in accordance with the direction and the distance calculated by the photoelectric sensor unit 52 and thereby and thereby extends or retracts the arm 54 depending on a result of calculation. A distal end of the extensible/retractable arm 54 is rotatably connected to a distal end 59 of the swing arm 49 by means of a pivot pin 56. A proximal end 57 of the swing arm 49 opposite to the distal end 59 is mounted on the movable plate 47 by means of a pivot pin 58 around which the swing arm 49 can swing in- and outward in the cross direction CD. The distal end 59 of the swing arm 49 is provided with a roll unit 63 specifically comprising first and second nip rolls 61, 62 arranged vertically as viewed in Fig. 4. The roll unit 63 comprises a bracket 64 fixed to the swing arm 49, a supporter 67 extending from this bracket 64 and supporting a first rotary shaft 66 at its opposite ends and a second rotary shaft 68 extending from the bracket 64 and supporting the second nip roll 62 at its opposite ends. The first nip roll 61 and the second nip roll 62 are free to rotate about the first rotary shaft 66 and the second rotary shaft 68, respectively, both in the machine direction MD and in the direction opposite to the machine direction MD.

The photoelectric sensor unit 52 shown in Fig. 4 has an upper arm 82 provided with a light emitter 82a extending in the cross section CD and a lower arm 83 provided with a light receiver 83a extending in the cross section CD. From the light receiver 83a, an output cord 83b extends toward the actuator 48. The unit 52 is adapted to detect the first side edge 31 of the first continuous web 21 indicated by imaginary line. In Figs. 2 and 3, a photoelectric sensor unit 152 in the second edge controller 42 of the edge controller 40 is shown as mounted on a support bar 151, but this photoelectric sensor unit 152 is not shown in Fig. 4.

With the edge controller 40 constructed in this manner, an actual position of the first side edge 31 detected by the photoelectric sensor unit 52 located downstream of the first edge controller 41 is compared with the predetermined position on which the first side edge 31 should fall. Operation of the actuator 48 based on such comparison causes the swing arm 49 connected with the extensible/retractable arm 54 to swing about the pivot pin 58 by a desired angle inward or outward in the cross direction CD, i.e., inward or outward in the transverse direction of the first continuous web 21. The roll unit 63 mounted on the distal end 59 of the swing arm 49 also swings as the swing arm 49 swings in this manner.

In the roll unit 63, the first rotary shaft 66 of the first nip roll 61 and the second rotary shaft 68 of the second nip roll 62 extend between the machine direction MD and the cross direction CD and normally extend so as to intersect the machine direction MD. In Figs. 2 and 3, the first nip roll 61 extending in the transverse direction of the first continuous web 21 after having slightly swung from the cross direction CD toward the machine direction MD is indicated by solid line while the first nip roll 61 having significantly swung outward in the transverse direction of the first continuous web 21 is indicated by imaginary line. The second nip roll 62 underlies the first nip roll 61 and therefore can not be seen in Figs. 2 and 3. The roll unit 63 functions to move the first side edge 31 and its vicinity of the first continuous web 21 nipped between the first and second nip rolls 61, 62 outward in the transverse direction as the swing arm 49 swings outward in the transverse direction of the first continuous web 21. In this course, the first and second nip rolls 61, 62 substantially falling on the cross direction as viewed in Figs. 2 and 3 swing so that their respective inner ends 61a, 62a put aside inward in the transverse direction of the first continuous web 21 move to downstream of their respective outer ends 61b, 62b put aside outward in the transverse direction of the first continuous web 21 as viewed in the machine direction MD. In other words, swing of the arm 49 outward in the transverse direction of the first continuous web 21 causes the rotary shafts 66, 68 of the first and second nip rolls 61, 62 to swing so that a tilt angle α of these rotary shafts 66, 68 relative to the machine direction MD gradually decreases. The movable plate 47 on which the first edge controller 41 is mounted is fixed at a position in the cross direction CD adjusted so that the first and second nip rolls 61, 62 can repetitively swing at any given time in the vicinity of the first side edge 31 of the first continuous web 21.

Similar to the first edge controller 41, the second edge controller 42 constituting the edge controller 40 comprises a movable plate 147 mounted on the base plate 46 so that its position in the cross direction CD may be adjusted, an actuator 148, a swing arm 149, a pivot pin 148, a roll unit 163 and a photoelectric sensor unit 152. Of these reference numerals, those added with 100 designate the components constituting the second edge controller 42. In the second edge controller 42, the roll unit 163 is placed in a mirror symmetric relationship with the roll unit 63 in the first edge controller 41 and the other components such as the actuator 148 and the swing arm 149 are mounted on the movable plate 147 in an appropriate layout allowing such mirror symmetric relationship between these roll units 63. 163. The arm 149 adapted to in- and outward swing on the basis of data supplied from the photoelectric sensor unit 152 adapted to detect an actual position of the second side edge 32. In response to swing of this arm 149 outward in the transverse direction of the first continuous web 21, the first and second nip rolls 161, 162 of the roll unit 163 swing in the same manner as in the case of the first and second nip rolls 61, 62 of the roll unit 63 so that so that their respective inner ends 161a, 162a put aside inward in the transverse direction of the first continuous web 21 move to downstream of their respective outer ends 161b, 162b put aside outward in the transverse direction of the first continuous web 21 as viewed in the machine direction MD with a tilt angle β of these nip rolls 161b, 162b relative to the machine direction MD gradually decreasing. The first and second edge controllers 41, 42 operate independently of each other.

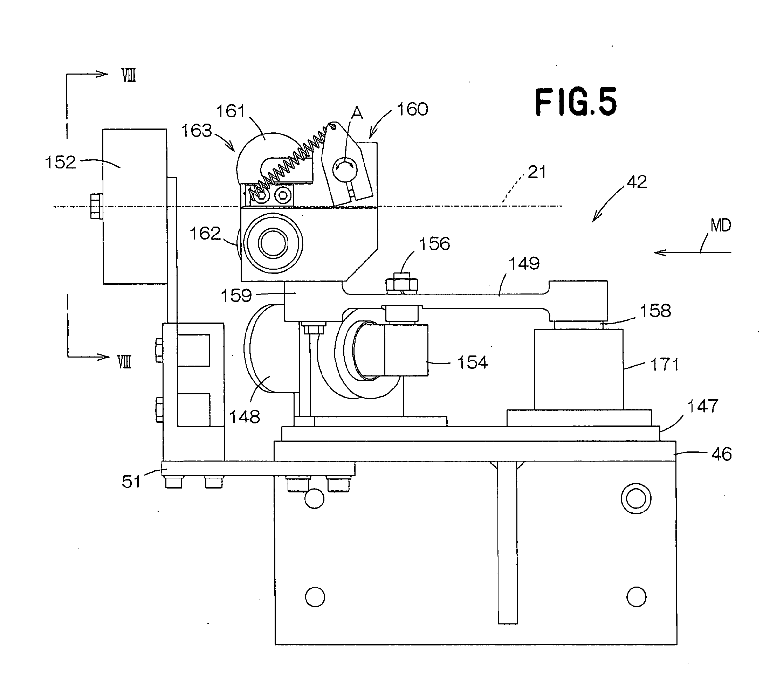

Fig. 5 is a sectional view taken along the line V-V in Fig. 3, showing profiles of the second edge controller 42 and the photoelectric sensor unit 152. In Fig. 5, the roll unit 163 is oriented in coincidence with the cross direction CD. In the second edge controller 42, the pivot pin 158 of the swing arm 149 lies upstream in the machine direction MD and rotatably mounted on a pedestal 171 which is, in turn, fixed to the movable plate 147. The swing arm 149 extends from upstream toward downstream in the machine direction MD and provided on its distal end 159 with the roll unit 163 fixed thereto. In the vicinity of the distal end 159, the extensible/retractable arm 154 is mounted on the swing arm 149 by means of the pivot pin 156 so that the arm 154 can rotate around the pivot pin 156. The roll unit 163 comprises the first nip roll 161 and the second nip roll 162. After having been nipped by these two rolls 161, 162, the first continuous web 21 horizontally runs through the photoelectric sensor unit 152 and further runs toward downstream in the machine direction MD as indicated by imaginary line.

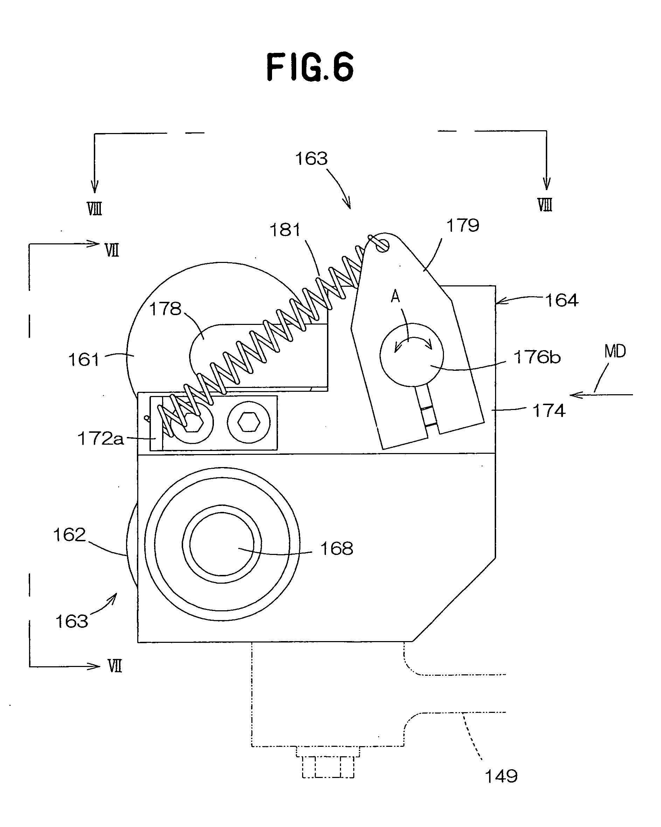

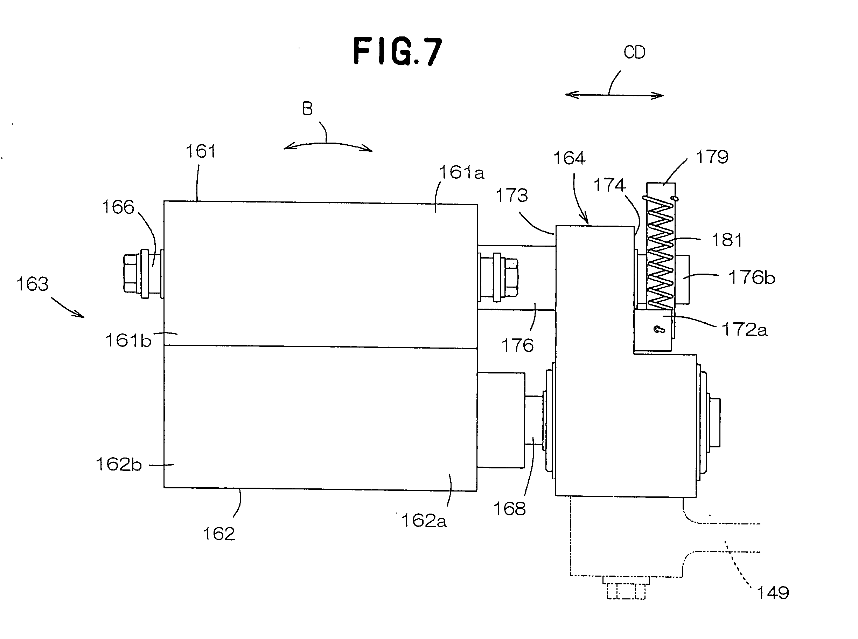

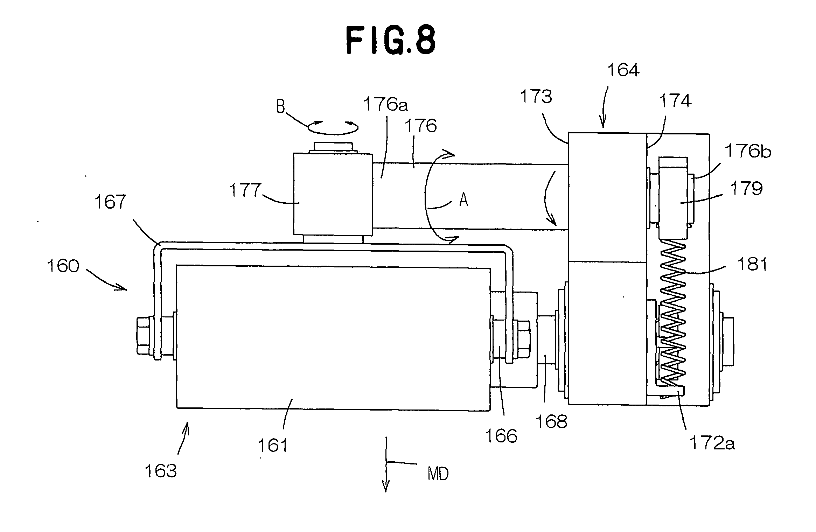

Fig. 6 is a scale-enlarged view showing the roll unit 163 Fig. 5, Fig. 7 is a sectional view taken along the line VII-VII, and Fig. 8 is a sectional view taken along the line VIII-VIII in Fig. 5, respectively. The roll unit 163 has a bracket 164 fixed to the swing arm 149. The bracket 164 has inner and outer surfaces 173, 174 placed aside inward and outward, respectively as viewed in the transverse direction of the first continuous web 21. From the inner surface 173, a cylindrical arm 176 horizontally extends and supports the first nip roll 161 and this arm 176 is provided on its inner end 176a with an inverted square U-shaped supporter 167 mounted thereon by means of coupling means 177 as will be seen in Fig. 8. The supporter 167 rotatably supports opposite ends of a first rotary shaft 166 of the first nip roll 161. Also from the inner surface 173, a second rotary shaft 168 supporting the second nip roll 162 horizontally extends and rotatably mounted on the bracket 164. From the outer surface 174 of the bracket 164, an outer end 176b of the cylindrical arm 176 extends and a plate member 179 is fixed around this outer end 176b. One end of a coil spring 181 operatively associated with the nip roll unit is anchored on the plate member 179 and the opposite end of the coil spring 181 is anchored on an angular plate 172a of the bracket 164.

In the roll unit 163 constructed in this manner, the bracket 164 swings in- and outward integrally with the swing arm 149 around the pivot pin 158. The first nip roll 161 is rotatable about the first rotary shaft 166 toward down- and upstream in the machine direction MD. The cylindrical arm 176 is supported by the bracket 164 and rotatable in two directions indicated by a double-headed arrow A (See Fig. 6). While the first nip roll 161 can be brought in contact with or spaced from the second nip roll 162 as the cylindrical arm 176 rotates, the first nip roll 161 is normally biased by the spring 181 to come in contact with the second nip roll 162. The first nip roll 161 is also rotatable around the coupling means 177 in the direction indicated by the double-headed arrow B (See Fig. 8) . The second nip roll 162 is supported by the bracket 164 by means of the second rotary shaft 168 and rotatable toward downstream as well as toward upstream in the machine direction MD. Swing of the first nip roll 161 in the directions indicated by the double-headed arrows A and B causes the vicinity of the second side edge 32 of the first continuous web 21 nipped between the first nip roll 161 and the second nip roll 162 to be held in close contact with these two rolls 161, 162 and thereby to follow in- and outward swinging of these two rolls 161, 162.

The first and second nip rolls 61, 62 of the roll unit 63 and the first and second nip rolls 161, 162 of the roll unit 163 tilt at angles of α and β relative to the machine direction MD when the roll units 63, 163 respectively nip the vicinity of the first side edge 31 and the vicinity of the second side edge 32 of the first continuous web 21. Consequently, a force tending to broaden a transverse dimension of the first continuous web 21 is exerted on the extent of this web 21 between the first and second side edges 31, 32 thereof. If the first continuous web 21 guided through the edge controller 40 has wrinkles, the force tending to broaden the web 21 functions to unwrinkled the web 21. When such effect is not required, the roll units 63, 163 may be previously swung in the respective directions in which the tilt angles α and β are appropriately decreased. The directions in which the pair of the first and second nip rolls 61, 62 and the pair of the first and second nip rolls 161, 162 are swung about the pivot pins 58, 158, respectively may be appropriately selected to move the vicinity of the first side edge 31 and the vicinity of the second side edge 32 nipped by these pairs of the nip rolls, respectively, together with these rolls outward as well as inward in the transverse direction of the first continuous web 21.

As will be understood from the aforementioned description, the method and the apparatus according to the present invention is primarily in that a swinging direction and a swinging extent (i.e., swinging angle) of the first and second nip roll pairs 61, 62; 161, 162 are linked with the respective positions of the first and second side edges 31, 32 of the first continuous web 21 detected by the photoelectric sensor units 52, 152, respectively. More specifically, the photoelectric sensor units 52, 152 are provided immediately downstream of the respective roll units 63, 163 so that a combined effect of the first and second nip roll pairs 61, 161; 161, 162 swinging in this direction and by this extent may be rapidly fed back to the respective actuators 48, 148 and thereby the first continuous web 21 may be rapidly and accurately controlled. It is also possible to ensure a rapid position-change of the first and second side edges 31, 32 by accelerating the servomotors' rotational velocities and thereby accelerating the swinging velocities of the roll units 63, 163.

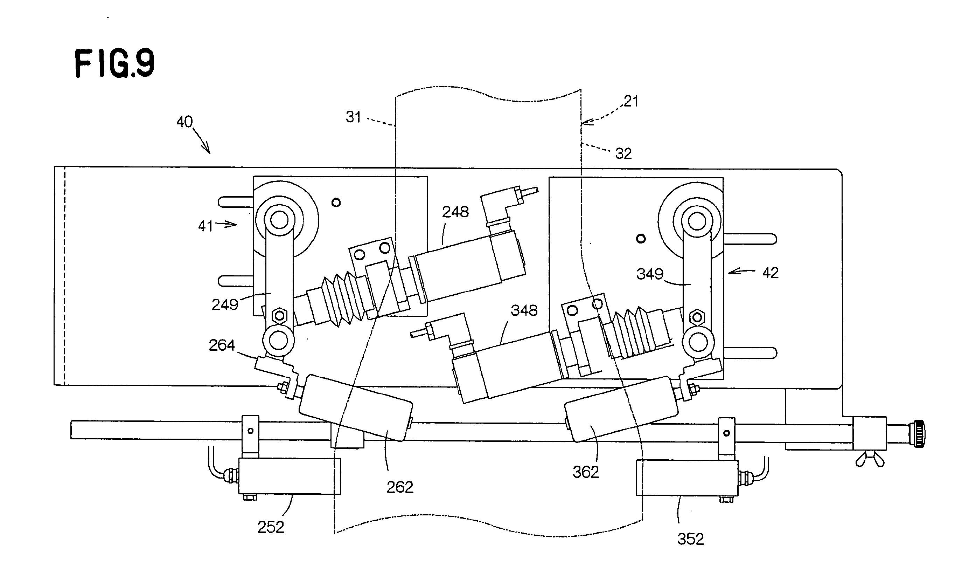

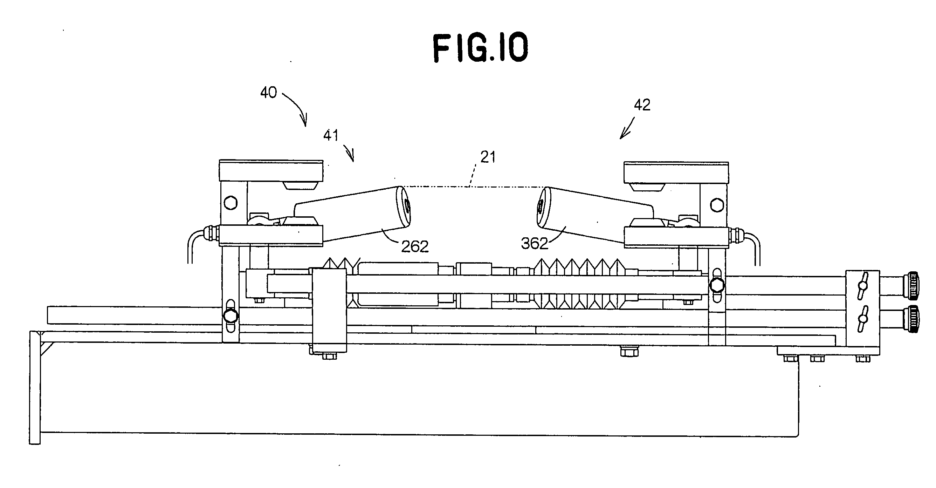

Fig. 9 is a view similar to Fig. 3, showing one preferred embodiment of the present invention and Fig. 10 is a view similar to Fig. 4, showing this embodiment. The edge controller 40 according to this embodiment is distinguished from that shown by Figs. 2 through 4 in that each of the first and second edge controllers 41, 42 comprises a lower roll 262, 362 adapted to come in contact with the lower surface of the first continuous web 21 and not an upper roll adapted to come in contact with the upper surface of the first continuous web 21. While the positions at which the lower rolls 262, 362 are provided are substantially the same as those at which the second nip rolls 62, 162 of Fig. 4 are provided, the first edge controller 41 according to the embodiment shown by Figs. 9 and 10 is distinguished from the previous embodiment shown by Fig. 4 in that a bracket 264 is mounted on a swing arm 249 so that the bracket 264 may swing about the arm 249. The lower roll 262 mounted on the bracket 264 extends inward in the transverse direction of the first continuous web 21 so as to describe a rising slope (See Fig. 10) as the bracket 264 appropriately swings. This is true for the lower roll 362 of the second edge controller 42. The first continuous web 21 is brought into a state as shown by Fig. 10 as these lower rolls 262, 362 are pressed against the vicinities of the first and second side edges 31, 32, respectively. Then, these rolls 262, 362 in- and outward swing together with the respective swing arms 249, 349 in the transverse direction of the first continuous web 21 and thereby guide the first and second side edges 31, 32 to the desired positions.. According to this embodiment also, the positions actually the first and second side edges 31, 32 passing after having been guided by the rolls 262, 362 are detected by photoelectric sensor units 252, 352 and actuators 248, 348 operate in response to data supplied from these sensor units 252, 352 so that the side edges 31, 32 may be guided to the predetermined positions more accurately. Slopes at which the lower rolls 262, 362 should tilt may be appropriately adjusted taking account of particular characteristics of the first continuous web 31 such as flexibility, elasticity and slipperiness.

While the invention has been described hereinabove on the basis of the embodiments in which the first and second side edges 31, 32 including the vicinities thereof of the first continuous web 21 are guided by the first and second end controllers 41, 42, respectively, to the desired positions, the invention may be implemented in a manner that only one of the side edges including the vicinity of this side edge is guided to the desired position. Without departing from the scope of the invention, an alternative arrangement is also possible in which the first and second nip rolls 61, 62 are shifted from each other in the machine direction MD, i.e., one of the nip rolls is located upstream and the other is located downstream in the machine direction MD. Factors such as material, type of surface finish, length and diameter may be appropriately selected depending on the manner in which the first continuous web 21 has been processed and coated with adhesive, if any. The roll may be formed from use of materials, for example, sponge rubber obtained from various types of rubber such as urethane rubber and silicone rubber or metallic material. The roll may be subjected to a surface treatment such as a satin-like finish to adjust a slipperiness relative to the first continuous web 21. Means for position-detection of the first and second side edges 31, 32 is not limited to the photoelectric sensor unit as illustrated and this photoelectric sensor unit may be replaced by the other optical means or physical means. While the present invention has been described with respect to the first continuous web 21 used for the disposable diaper 1, the invention is applicable also to the second continuous web 22 and the other types of continuous web used for articles other than the diaper 1.

According to the present invention, the rotatable rolls used to come in contact with the vicinities of the side edges of the web continuously running in the machine direction and thereby to guide these side edges in the cross direction are adapted to swing in- and outward in the transverse direction of the web wherein the direction and the extend (angle) of swing relative to the machine direction are determined on the basis of the detected positions of the respective side edges of the running web. According to the present invention, the angle of the rolls relative to the machine direction can be adjustably varied to vary a force tending to broaden the web and at the same time the positions of the side edges of the web can be forcibly and quickly varied by oscillating the rolls. In this way, the side edges of the web can be easily and accurately guided to the predetermined position.

## Claims

1. A method for guiding a web continuously running in a machine direction and having a pair of side edges opposed to each other in a cross direction orthogonal to said machine direction so that at least one of said side edges is guided to a predetermined position in said cross direction by putting roll means rotating about a shaft extending in a direction defined between said machine direction and said cross direction in contact with a vicinity of at least one of said side edges, said method comprising the steps of:
mounting an arm having a proximal end and a distal end opposed to said proximal end at said proximal end on a pivot pin adapted to rotate back-and-forth so that said arm swings around said pivot pin in- and outward as viewed in said cross direction;
supporting said shaft of said roll means by said distal end of said arm so that said roll means also swing about said pivot pin in- and outward;
detecting, at downstream of said roll means, a position of said one side edge after this side edge has been guided; and
driving said arm by driving means adapted to operate on the basis of a difference between said position detected in this manner and said desired position so that said arm swings in- or outward in said cross direction by a predetermined angle.

2. The method according to Claim 1, wherein said driving means comprises an extensible/retractable arm operatively associated with a servomotor adapted to rotate in a direction and at a rotational frequency depending on the difference between said detected position and said desired position and thereby to extend or retract said extensible/retractable arm so that said side edge is guided to said desired position.

3. The method according to Claim 1 or 2, wherein said roll means is put in contact with at least one side of said web.

4. The method according to Claim 1 or 2, wherein said roll means comprises a pair of rolls extending in parallel to each other and adapted to nip at least said one side edge and the vicinity thereof.

5. The method according to any one of Claims 1, 2, 3 and 4, wherein said roll means are provided in vicinities of a pair of said side edges to guide these side edges to respective said desired positions.

6. An apparatus for guiding a web continuously running in a machine direction and having a pair of side edges opposed to each other in a cross direction orthogonal to said machine direction so that at least one of said side edges is guided to a predetermined position in said cross direction by putting roll means rotating about a shaft extending in a direction defined between said machine direction and said cross direction in contact with a vicinity of at least one of said side edges, said apparatus further comprising:
said shaft of said roll means being supported on a distal end of an arm which has a proximal end and said distal end opposed to said proximal end;
said proximal end of said arm being mounted on a pivot pin adapted to rotate back-and-forth so that said arm swings about said pivot pin in- and outward as viewed in said cross direction;
detector means being provided downstream of said roll means in said machine direction to detect a position of said one side edge immediately after said one side edge has been guided; and
said arm being operatively associated with driving means adapted to operate on the basis of a difference between said position detected by said detector means and said desired position so that said arm swing in- or outward in said cross direction by a desired angle.

7. The apparatus according to Claim 6, wherein said driving means comprises an extensible/retractable arm operatively associated with a servomotor.

8. The apparatus according to Claim 6 or 7, wherein said roll means is put in contact with at least one side of said web.

9. The apparatus according to Claim 6 or 7, wherein said roll means comprises a pair of rolls extending in parallel to each other and adapted to nip at least said one side edge and the vicinity thereof.

10. The apparatus according to any one of Claims 6, 7, 8 and 9, wherein said roll means are provided in vicinities of a pair of said side edges to guide these side edges to respective said predetermined positions.
